# EUROPEAN PATENT SPECIFICATION

(11) **EP 2 643 347 B1**
(45) Date of publication and mention of the grant of the patent: **27.12.2017**
(21) Application number: 11788142.5
(22) Date of filing: 24.11.2011
(51) Int. Cl.: A61K 39/00, C07K 14/435, C07K 14/755, C07K 16/00, C07K 2/00, C07K 14/415, C12N 9/64

(54) **MODULATION OF ANTIGEN IMMUNOGENICITY BY DELETING EPITOPES RECOGNIZED BY NKT CELLS**
MODULATION DER ANTIGENIMMUNOGENITÄT MITTELS ENTFERNUNG VON DURCH NKT-ZELLEN ERKANNTEN EPITOPEN
MODULATION DE L'IMMUNOGÉNICITÉ DES ANTIGÈNES PAR LA DÉLÉTION D'ÉPITOPES RECONNUS PAR LES CELLULES NKT

(30) Priority: 25.11.2010 EP 10192568
(43) Date of publication of application: 02.10.2013
(73) Proprietor: IMNATE SARL, 8041 Strassen (LU)
(72) Inventor: SAINT-REMY, Jean-Marie, 1390 Grez-Doiceau (BE)
(74) Representative: Office Kirkpatrick
(86) International application number: PCT/EP2011/070911
(87) International publication number: WO 2012/069575

(56) References cited:
- WO-A2-02/19968
- WO-A2-2010/065544
- TEXIER C ET AL: "HLA-DR restricted peptide candidates for bee venom immunotherapy.", JOURNAL OF IMMUNOLOGY (BALTIMORE, MD. : 1950) 15 MAR 2000 LNKD- PUBMED:10706708, vol. 164, no. 6, 15 March 2000 (2000-03-15), pages 3177-3184, XP002640295, ISSN: 0022-1767
- CASTANO A R ET AL: "PEPTIDE BINDING AND PRESENTATION BY MOUSE CD1", SCIENCE, AMERICAN ASSOCIATION FOR THE ADVANCEMENT OF SCIENCE, WASHINGTON, DC; US, vol. 269, no. 5221, 14 July 1999 (1999-07-14), pages 223-226, XP008065110, ISSN: 0036-8075, DOI: 10.1126/SCIENCE.7542403 cited in the application
- MATSUDA J L ET AL: "CD1d-restricted iNKT cells, the 'Swiss-Army knife' of the immune system", CURRENT OPINION IN IMMUNOLOGY, ELSEVIER, OXFORD, GB, vol. 20, no. 3, 1 June 2008 (2008-06-01), pages 358-368, XP022762945, ISSN: 0952-7915, DOI: 10.1016/J.COI.2008.03.018 [retrieved on 2008-05-22]
- GODFREY DALE I ET AL: "Raising the NKT cell family", NATURE IMMUNOLOGY, NATURE PUBLISHING GROUP, GB, vol. 11, no. 3, 1 March 2010 (2010-03-01), pages 197-206, XP002633198, ISSN: 1529-2908, DOI: 10.1038/NI.1841 [retrieved on 2010-02-07]
- LEE D J ET AL: "INDUCTION OF AN ANTIGEN-SPECIFIC, CD1-RESTRICTED CYTOTOXIC T LYMPHOCYTE RESPONSE IN VIVO", THE JOURNAL OF EXPERIMENTAL MEDICINE, ROCKEFELLER UNIVERSITY PRESS, US, vol. 187, no. 3, 2 February 1998 (1998-02-02), pages 433-438, XP002908476, ISSN: 0022-1007, DOI: 10.1084/JEM.187.3.433 cited in the application
- TANGRI SHABNAM ET AL: "Presentation of peptide antigens by mouse CD1 requires endosomal localization and protein antigen processing", PROCEEDINGS OF THE NATIONAL ACADEMY OF SCIENCES OF THE UNITED STATES OF AMERICA, vol. 95, no. 24, 24 November 1998 (1998-11-24), pages 14314-14319, XP002670194, ISSN: 0027-8424
- SWAROOP M ET AL: "Mutagenesis of a potential immunoglobulin-binding site enhance", JOURNAL OF BIOLOGICAL CHEMISTRY, AMERICAN SOCIETY FOR BIOCHEMISTRY AND MOLECULAR BIOLOGY, US, vol. 272, no. 39, 1 January 1997 (1997-01-01), pages 24121-24124, XP002988831, ISSN: 0021-9258, DOI: 10.1074/JBC.272.39.24121

## Description

### Field of the invention

The present disclosure relates to peptides or polypeptides to decrease natural immunogenicity or induce tolerance and their use in preventing immune responses elicited towards allofactors, towards viral vectors used for gene therapy or gene vaccination, towards environmental antigens used in food or feed or to which subjects are exposed by inhalation or contact, or to decrease side effects associated with vaccination by allergens or infectious agents.

### Background of the invention

In many instances, administration of proteins for therapeutic purposes results in immune response against the therapeutic agents, which precludes any further administration of said therapeutic agent. Examples of this are provided by administration of factor VIII of the coagulation pathway in subjects affected by hemophilia A: about a third of treated subjects produce anti-factor VIII antibodies inhibiting the function of factor VIII. Administration of enzymes such as alpha-galactosidase in subjects suffering from deficiency in glycogen metabolism is likewise followed by an immune response against the therapeutic agent precluding any further administration. A third example is provided by antibodies used as therapeutic agents and directed towards lymphocyte surface molecule, cytokines or cytokine receptors. Overall, it is highly desirable to find new therapeutic approaches which would prevent immunization against therapeutic agents.

The use of viral vectors for gene therapy or gene vaccination is severely restricted by the fact that such viral vectors elicit an immune response which results in rapid elimination of cells transduced by the vector and rapid loss of transgene expression. Viral vectors elicit activation of the innate and adaptive immune response with variable degree of intensity depending of the nature of the viral vector. Thus, adenovirus vectors strongly stimulate innate immune responses followed by an adaptive immune response. Adeno-associated viral vectors trigger a weaker innate response but elicit antibody production. The potential of both gene therapy and gene vaccination is huge. A therapeutic approach by which the innate and/or the adaptive response towards viral vectors would represent a highly significant progress in the field. Many subjects suffer from response elicited against proteins to which they are naturally exposed. Allergens, either airborne or from food, elicit reactions such as allergic rhinitis and asthma, urticaria, eczema and anaphylactic reaction. The reason as to why said subjects present such reactions is only partially elucidated. It would be of much benefit to reduce the natural immunogenicity of proteins a diverse as food allergens, wheat causing celiac disease or products such as enzymes to which subjects are exposed for professional reasons.

In vaccination strategies for either allergic diseases or against infectious agents, the therapeutic efficacy is often limited by side effects which are elicited by the proinflammatory properties of allergens or infectious agents. Such inflammatory effects preclude the use of higher doses of vaccines, and therefore of vaccine efficacy, and orientate the immune response towards unwanted cellular response, such as delayed-type reaction, and production of antibodies of an isotype which is not optimal for the condition considered. A better control of inflammation in vaccination would offer a much more efficient modulation of the immune response whilst reducing side effects related to inflammation.

Natural killer T (NKT) cells constitute a distinct lineage of non-conventional T lymphocytes that recognize antigens presented by the non-classical MHC complex molecule CD1d. Two subsets of NKT cells are presently described. Type 1 NKT cells, also called invariant NKT cells (iNKT), are the most abundant. They are characterized by the presence of an alpha-beta T cell receptor (TCR) made of an invariant alpha chain, Valpha14 in the mouse and Valpha24 in humans. This alpha chain is associated to a variable though limited number of beta chains. Type 2 NKT cells have an alpha-beta TCR but with a polymorphic alpha chain. However, it is apparent that other subsets of NKT cells exist, the phenotype of which is still incompletely defined, but which share the characteristics of being activated by glycolipids presented in the context of the CD1d molecule.

NKT cells typically express a combination of natural killer (NK) cell receptor, including NKG2D and NK1.1. NKT cells are part of the innate immune system, which can be distinguished from the adaptive immune system by the fact that they do not require expansion before acquiring full effector capacity. Activation of NKT cells results in various effects. Such cells release preformed mediators, including a large array of cytokines (including interleukin (IL)-4, interferon (IFN)-gamma, II-21 and IFN-alpha) which provide help to B cells for the production of antibodies and it has been suggested that the release of cytokines could also influence CD4+ T cells (Burrows et al Nature Immunology 2009, 10: 669-671). In the context of the present invention, the prevention of both B cell activation and of major histocompatibility (MHC) class II-restricted CD4+ T cells activation is deemed to play a role in reducing or abolishing protein immunogenicity.

The recognition unit for NKT cells, the CDld molecule, has a structure closely resembling that of the MHC class I molecule, including the presence of beta-2 microglobulin. It is characterized by a deep cleft bordered by two alpha chains and containing highly hydrophobic residues, which accepts lipid chains. The cleft is open at both extremities, allowing to accommodate longer chains. The canonical ligand for CDld is the synthetic alpha galactosylceramide (alpha GalCer). However, many natural alternative ligands have been described, including glyco- and phospholipids, the natural lipid sulfatide found in myelin, microbial phosphoinositol mannoside and alpha-glucuronosylceramide. The present consensus (see reviews, such as Matsuda et al, Current Opinion in Immunology 2008, 20:358-368 and Godfrey et al, Nature reviews Immunology 2010, 11: 197-206) is that CDld binds only ligands containing lipid chains, or in general a common structure made of a lipid tail which is buried into CDld and a sugar residue head group that protrudes out of CD1d.

Peptides are not deemed to be able to activate NKT cells through presentation by CD1d. It was, however, suggested that long hydrophobic peptides containing bulky aminoacid residues could bind to CDld (Castano et al, Science 1995, 269: 223-226). Observations carried out using phage display libraries expressing random sequence peptides with no defined physiological relevance, allowed establishing a theoretical consensus motif (Castano et al, Science 1995, 269: 223-226 and see below).

In fact, Castano et al show that the cells which are activated are CD8+ T cells, namely MHC class I restricted cells, and not NKT cells. These findings teach the one skilled in the art that there is no evidence that hydrophobic peptides are presented by CD1d molecules. The physiological relevance of the claims made by Castano et al was further questioned due to the inability to elicit NKT cells under conventional immunization protocols (Matsuda et al, Current Opinion in Immunology 2008, 20:358-368 and Brutkiewicz Journal of Immunology 2006, 177: 769-775). Artificial systems such as immunization with cells transfected to overexpress CDld and loaded in vitro with an ovalbumin-derived peptide were able to elicit NKT cells. Likewise, intradermal immunization with plasmid DNA together with murine CD1d and costimulatory molecules induce cytolytic CD1d-restricted T cells (Lee et al, Journal of Experimental Medicine 1998, 187: 433-438). Hydrophobic peptides containing a structural motif made of an aromatic residue in position P1 and P7, and an aliphatic chain in position P4 were claimed by Castano et al (Science 269: 223, 1995) to contain a core motif for CDld binding epitopes. As described above, the conclusions reached by Castano et al are not supported by data.

Swaroop M et al. "Mutagenesis of a potential immunoglobulin-binding protein-binding site enhances secretion of coagulation factor VIII", J. Biol. Chem., 1997, Vol. 272, pp 24121-24124, disclose the mutation F309S in factor VIII so as to increase its secretion. However, this publication does not disclose any effect on NKT cell activation.

We made the unexpected finding that peptides encompassing a hydrophobic aminoacid sequence are in fact capable of eliciting activation of NKT cells.

If epitopes from proteins administrated for therapeutic purposes, or to which subjects are normally exposed, or when gene therapy or gene vaccination is carried out, or administered in the context of vaccination for allergic or infectious diseases bind to CD1d and thereby activate NKT cells, then alteration of said proteins by mutations and/or deletions to eliminate said epitopes would be highly desirable to prevent immunogenicity.

Identification of such epitopes followed by mutation, addition or deletion of aminoacids to prevent activation of NKT cells forms the basis of the present invention.

### Summary of the invention

The present invention is as defined in the claims and disclosed herein is the use of peptides or polypeptides for the treatment of immune responses elicited towards an allofactor, being Factor VIII, in a subject by preventing such immune response towards said allofactor. Disclosed herein is the use of peptides or polypeptides for the treatment of immune responses elicited towards viral vectors used for gene therapy or gene vaccination in a subject by preventing such immune response towards said viral vectors. Disclosed herein is the use of peptides or polypeptides made by genetically-modified organisms for the prevention in a subject of immune responses elicited by exposure to natural proteins. Described herein is the use of peptides or polypeptides for vaccination purposes when activation of innate immunity is detrimental.

The present invention also relates to methods to identify proteins which carry CD1d binding epitopes and to eliminate such epitopes by aminoacid substitution or deletion.

We made the unexpected finding that a significant proportion of peptides or polypeptides carried aminoacid sequences which allow them to bind and to be presented by CDld determinants for activation of natural killer T (NKT) cells. Activation of such cells results in release of cytokines and, in some cases, in acquisition or increase of cytolytic properties.

The present invention relates in one aspect to SEQ.ID.NO:4, a polypeptide used as an allofactor, Factor VIII, which has been modified to eliminate at least two hydrophobic amino acid residues involved in the formation of an epitope recognized by NKT cells. Described herein is the use of at least one isolated peptide or polypeptide used as a viral vector for gene therapy or gene vaccination, which has been modified to eliminate at least one hydrophobic amino acid residue involved in the formation of an epitope recognized by NKT cells, as a medicament for preventing in a subject immune responses to said viral vectors. Described herein is the use of at least one isolated peptide or polypeptide produced by a genetically-modified organism, said peptide or polypeptide being modified to eliminate at least one hydrophobic amino acid residue involved in the formation of an epitope recognized by NKT cells, as a medicament for preventing in a subject immune responses to natural exposure to said peptides or polypeptides. Described herein is the use of at least one isolated peptide or polypeptide used as a vaccine, which has been modified to eliminate at least one hydrophobic amino acid residue involved in the formation of an epitope recognized by NKT cells, as a medicament for preventing in a subject an unwanted or inappropriate immune response to said vaccine. Described herein is the use of at least one isolated peptide or polypeptide used as an allofactor, a viral vector, a genetically-modified organism or a vaccine, which has been modified to eliminate at least one hydrophobic amino acid residue involved in the formation of an epitope recognized by NKT cells, as a medicament for preventing in a subject activation, cytokine production, cytolytic activity and suppressive activity on adaptive immune responses carried by CD4+ NKT cells in said subject. Described herein are hydrophobic peptides or polypeptides encompassing at least one CD1d-restricted T cell epitope, in which aminoacids positioned as anchoring residues to CD1d are replaced by alternative aminoacids, or deleted, which results in a loss or significant reduction of binding to CD1d and thereby of NKT cell activation.

The structure of the CDld molecule indicates that hydrophobic aminoacid residues are required to occupy the two hydrophobic pockets located at the extremities of the CD1d cleft and that an aliphatic residue should occupy the position in the middle of the cleft. Therefore, as a general example of CDld binding sequence, the motif [FW]-xx-[ILM]-xx-[FWTH] can be used in which [FW] indicates that either F or W can occupy the first anchoring residue (PI), that the P4 position can be occupied by either I, L or M and that P7 can be occupied by F, W, T or H. x in this general model motif stands for any aminoacid. It should be clear for the one skilled in the art that various combinations of these aminoacid residues are possible. In a particular embodiment the general model motif can be presented as a reverted sequence such as [FWTH]-xx-[ILM]-xx-[FW]. Described herein are peptides or polypeptides wherein F, W, T, H or Y in positions P1 and P7 are replaced by a non-natural amino acid (for example a D-aminoacid) or by an organic compound.

The present invention also relates to methods for identifying peptides or polypeptides activating NKT cells and eliminates such activation by altering CD1d binding epitopes by substitution or deletion of aminoacids. Said methods comprise the steps of incubating said peptide or polypeptide with cells carrying CD1d, followed by addition of a population of polyclonal NKT cells and determination of activation of said NKT cells.

The method of the present invention further encompasses isolated viral vectors characterized in that they comprise at least one peptide or polypeptide of an allofactor modified by substitution or deletion of at least one hydrophobic aminoacid, or at least one peptide or polypeptide from an allergen or from an infectious agent modified by substitution or deletion of at least one hydrophobic aminoacid residue. It should be understood that the viral vector itself may also be modified by substitution or deletion of hydrophobic aminoacid residues.

### Definitions

The term **"peptide"** when used herein refers to a molecule comprising an amino acid sequence of between 2 and 200 amino acids, connected by peptide bonds, but which can in a particular embodiment comprise non-amino acid structures (like for example a linking organic compound). Peptides according to the invention can contain any of the conventional 20 amino acids or modified versions thereof, or can contain non-naturally occurring amino acids incorporated by chemical peptide synthesis or by chemical or enzymatic modification. The term **"polypeptide"** when used herein refers to generally longer peptides or proteins.

The term **"epitope"** when used herein refers to one or several portions (which may define a conformational epitope) of a protein which is/are specifically recognized and bound by an antibody or a portion thereof (Fab', Fab2', etc.) or a receptor presented at the cell surface of a B or T cell lymphocyte, and which is able, by said binding, to induce an immune response. The term **"antigen"** when used herein refers to a structure of a macromolecule comprising one or more hapten(s) and/or comprising one or more T cell epitopes. Typically, said macromolecule is a protein or peptide (with or without polysaccharides) or made of proteic composition and comprises one or more epitopes; said macromolecule can herein alternatively be referred to as **"antigenic protein"** or **"antigenic peptide".**

The term **"allergen"** refers to a specific subset of antigen characterized by its capacity to elicit antibodies of the IgE isotype in predisposed individuals.

The term **"T cell epitope"** or **"T-cell epitope"** in the context of the present invention refers to a dominant, sub-dominant or minor T cell epitope, i.e., a part of an antigenic protein that is specifically recognized and bound by a receptor at the cell surface of a T lymphocyte. Whether an epitope is dominant, sub-dominant or minor depends on the immune reaction elicited against the epitope. Dominance depends on the frequency at which such epitopes are recognized by T cells and able to activate them, among all the possible T cell epitopes of a protein. In particular, a T cell epitope is an epitope bound by MHC class I or MHC class II molecules.

The term **"NKT cell epitope"** refers to a part of an antigenic protein that is specifically recognized and bound by a receptor at the cell surface of a T lymphocyte. In particular, a NKT cell epitope is an epitope bound by CDld molecules.

The term **"CD4+ effector cells"** refers to cells belonging to the CD4-positive subset of T-cells whose function is to provide help to other cells, such as, for example B-cells. These effector cells are conventionally reported as Th cells (for T helper cells), with different subsets such as Th0, Th1, Th2, and Th17 cells.

The term **"NKT cells"** refers to cells of the innate immune system characterized by the fact that they carry receptors such as NK1.1 and NKG2D, and recognize epitopes presented by the CDld molecule. In the context of the present invention, NKT cells can belong to either the type 1 (invariant) or the type 2 subset.

The **"CD1d molecule"** refers to a non-MHC derived molecule made of 3 alpha chains and an anti-parallel set of beta chains arranged into a deep hydrophobic groove opened on both sides and capable of presenting lipids, glycolipids or hydrophobic peptides to NKT cells.

The term **"immune disorders"** or **"immune diseases"** refers to diseases wherein a reaction of the immune system is responsible for or sustains a malfunction or non-physiological situation in an organism. Immune disorders in the context of the present invention refer to pathology induced by infectious agents and tumor surveillance.

The term **"allofactor"** refers to a protein, peptide or factor (i.e. any molecule) displaying polymorphism when compared between two individuals of the same species, and, more in general, any protein, peptide or factor that induces an (alloreactive) immune response in the subject receiving the allofactor. By extension, allofactors also include genetically-modified proteins used for feeding.

The term **"alloantigen"** or **"allograft antigen"** when used herein refer to an antigen derived from (shed from and/or present in) a cell or tissue which, when transferred from a donor to a recipient, can be recognized and bound by an antibody of B or T-cell receptor of the recipient. Alloantigens are typically products of polymorphic genes. An alloantigen is a protein or peptide which, when compared between donor and recipient (belonging to the same species), displays slight structural differences. The presence of such a donor antigen in the body of a recipient can elicit an immune response in the recipient. Such alloreactive immune response is specific for the alloantigen.

### Detailed description of the invention

Described herein are ways to prevent, in a subject, an immune response towards allofactors, towards viral vectors used for gene therapy or gene vaccination, towards proteins used for food or feed, towards proteins to which said subject is exposed by inhalation or by stings, or to prevent, in a subject, an undesirable activation of innate immunity in the use of vaccines towards allergens or infectious agents.

In particular, ways to prevent the expansion and functional activity of CD4+ NKT cells are disclosed. Such cells are usually classified into two distinct subsets, namely type 1 for NKT cells carrying an invariant TCR alpha chain (Valpha14 in the mouse, Valpha24 in humans), or type 2 NKT cells which present with a diverse alpha chain repertoire. However, recent evidence has suggested that alternative subsets of NKT cells which do not fit in the type 1 or type 2 category. It is the purpose of the present disclosure to include these non conventional NKT cells, provided they carry the CD4 co-receptor. Upon presentation of an antigen bound to CD1d, NKT cells are rapidly activated and secrete a number of cytokines thought to be determinant to influence other cells from both the innate and adaptive immune systems. In some circumstances, said activated NKT cells acquire or increase cytotoxic properties. In yet additional circumstances, said activated NKT cells suppress or reduce the elicitation of an adaptive immune response by interaction with class II-restricted CD4+ T cells.

In the context of the present invention, we made the unexpected observation that peptides can be presented by the CDld molecule. A characteristic of the CDld molecule is that it is made of two anti-parallel alpha chains forming a cleft sitting atop of a platform made of two anti-parallel beta chains. The cleft is narrow and deep and accept only hydrophobic residues, classically deemed to be only lipids. The cleft can accommodate a sequence of 7 aminoacids characterized as a hydrophobic residue in position (P)1 and 7, and an aliphatic residue in P4. P1 is an obligate hydrophobic residue, such as F, W, H or Y. However, P7 is permissive and can contain alternative residues provided they are not polar. Residues in P4 are preferably aliphatic but are optional. The sequence for a CD1d binding motif is therefore [FWTHY]-X₂X₃-[ILMV]-X₅X₆-[FWTHY]. It should however be clear for those skilled in the art that the motif is symetrical and that P7 can be considered as P1, and P1 can be considered as P7. Peptides and polypeptides considered for application of the present invention are defined according to their capacity to activate NKT cells by presentation into CD1d molecule. Hydrophobic peptides or polypeptides capable of activating NKT cells and, consequently, carrying a CD1d-binding motif are found in allofactors, viral vectors, proteins used for food or feed, proteins to which said subject is exposed by inhalation or by stings, genetically-modified proteins and allergens, thereby endowing said allofactor, viral vector, genetically-modified protein or allergen with the capacity to activate CD4+ NKT cells.

The present invention relates to the production of peptides or polypeptides containing CDld binding motif(s), which confer them with the capacity to activate NKT cells and which are modified by substitution of hydrophobic residues in P1 and/or P7, with, optionally, substitution or deletion of aliphatic residues in P4, or any combination of these, which results in a loss or significant reduction of the capacity of peptides or polypeptides to bind to CDld and thereby results in a loss or significant reduction of said peptides or polypeptides to activate NKT cells.

In a more particular embodiment, F, W, T, H or Y in positions P1 and/or P7 are replaced by a non-hydrophobic aminoacid residue, or, optionally, I, L, M or V in position P4 is replaced by a non-aliphatic residue, or any combination of these.

In yet another particular embodiment, hydrophobic residues located in position P1 and/or P7, or, optionally, aliphatic residues located in P4, or any combination of these, are replaced by at least one non-natural aminoacid different from non-natural F, W, T, H, Y, or by a non-aromatic organic compound.

In yet another particular embodiment at least one aminoacid is added within the CDld binding motif, in any location within the P1 to P7 sequence, which disrupts the motif, prevents its capacity to bind to CDld and thereby its capacity to activate NKT cells.

In a preferred embodiment, non-natural aminoacids are D-aminoacids.

The present invention also relates to the production of peptides or polypeptides containing CDld binding motif(s), which confer them with the capacity ot activate NKT cells, and which are modified by deletion of hydrophobic residues in P1 and/or P7, or, optionally, by deletion of aliphatic residues in P4, or any combination of these, which results in a loss or significant reduction of the capacity of peptides or polypeptides to bind to CDld and thereby results in a loss or significant reduction of said peptides or poylpeptides to activate NKT cells.

Upon administration to a subject, such peptides or polypeptides are not loaded on CDld and thereby are prevented from activating NKT cells.

In a further aspect, disclosed herein is the use of at least one isolated peptide or polypeptide comprising at least one substitution or deletion of F, W, T, H or Y in positions P1 or P7 for preventing in a subject an immune response towards allofactor administration, viral vector administration, proteins to which said subject is exposed by food, feed, systemic or inhalation route, or allergens or infectious agents used for vaccination purposes. Disclosed herein is the use of at least one isolated peptide or polypeptide comprising at least one substitution or deletion of F, W, T, H or Y in positions P1 or P7 for preventing in a subject the activation of NKT cells towards allofactor administration, viral vector administration, proteins to which said subject is exposed by food, feed, systemic or inhalation route, or some allergens or infectious agents used for vaccination purposes. Disclosed herein is the use of at least one isolated peptide or polypeptide comprising at least one substitution or deletion of F, W, T, H or Y in positions P1 or P7 as a medicament for preventing in a subject an immune response towards allofactor administration, viral vector administration, proteins to which said subject is exposed by food, feed, systemic or inhalation route, genetically modified peptides or polypeptides or some allergens or infectious agents used for vaccination purposes.

The number of CD1d binding motifs when present in a peptide or polypeptide, is very limited. Examples of such peptides or polypeptides are provided below. Typically a polypeptide presents one to five of these motifs.

An additional advantage of the present invention is that the CD1d molecule presents a very limited degree of polymorphism. It is therefore obvious for the one skilled in the art that the same aminoacid substitutions or deletions according to the present invention provide peptides or polypeptides useful for all or a large majority of subjects. This is in sharp contrast with peptide or polypeptide motifs binding to MHC class II molecules, wherein a large number of peptides can be delineated which contain the appropriate sequence. This is due to the minimum constraints imposed to MHC class II binding peptides and to the large polymorphism of class II molecules.

Peptides and polypeptides which are the object of the present invention are identified as follows:
(1) a peptide or polypeptide aminoacid sequence is evaluated for the presence of at least one CD1d motif containing an hydrophobic residue in P1 and P7, and an aliphatic residue in P4. The sequence [FWTHY]- X₂X₃-[ILMV]-X₅X₆-[FWTHY] is used for using algorithms well known in the art such as scan prosite on expasy website.
   This general sequence should be considered as a tool to help identifying which sequence(s) in said peptide or polypeptide contain a motif which could enable said peptide or polypeptide to activate NKT cells.
(2) the capacity of the peptide or polypeptide to bind to CD1d and to activate NKT cells is tested in vitro using a cell line expressing the CD1d molecule. Examples of such cell lines are known in the art (for instance JAWS2 cells). In a preferred embodiment, the cell line is not presenting MHC class II molecules and is transduced for hyperexpression of CDld using a viral vector containing the DNA sequence of CD1d or any other means known in the art to introduce a gene in a cell. Methods for cell transduction are known in the art. The cell line is loaded in culture with the peptide or polypeptide, or with a synthetic peptide encompassing the corresponding sequence. Such synthetic peptides are easily produced by synthesis, using for instance the fmoc solid phase synthesis well known in the art. Efficient presentation of the peptide, polypeptide or corresponding synthetic peptide by the CD1d molecule is then evaluated by measuring the activation of NKT cells. Such cells can be obtained from peripheral blood by, for instance, magnetic sorting and maintained in culture with stimulants such as alpha-gal-ceramide, in the presence of cytokines such as IL-2, IL-15 or IL-7. These methods are described in the art (see for instance Godfrey et al, Nature Reviews. Immunology 2010, 11: 197-206). Activation of NKT cells is assessed using methods such as evaluation of cytokine production.
   Alternatively, peptides actually presented by APC in CDld molecules can be eluted and separated by various chromatography methods. Full description of such methodology will be found in Scott et al, Immunity, 12: 711-720, 2000. Said peptides are then sequenced to identify which aminoacid residues are located in P1 and P7.
   Alternatively, said synthetic peptides can be loaded on tetramers of the CDld molecule to detect NKT cells specific for such peptide. One possibility is to use fluorescence-labeled tetramers and detection using a fluorescence-activated cell sorting system (facs).
(3) the aminoacid sequences identified as being able to activate NKT cells and, optionally, identified by algorithms, are then modified by either substitution or deletion. In a preferred embodiment, F, W, T, H or Y in positions P1 and/or P7 are replaced by at least one aminoacid different from F, W, T, H, Y. Natural aminoacids can be modified by post-transcriptional modifications or substituted with chemical groups such as methyl groups. In another preferred embodiment, F, W, T, H or Y in positions P1 and/or P7 are replaced by any suitable alternative non-natural aminoacid. Examples of non-natural aminoacid residues are D-aminoacids. In yet another embodiment, F, W, T, H or Y in positions P1 and/or P7 are replaced by at least one aminoacid different from F, W, T, H, Y. In another preferred embodiment, F, W, T, H or Y in position P1 is replaced by at least one aminoacid different from F, W, T, H, Y, by any suitable alternative non-natural aminoacid or by a non-aromatic organic compound. Such aminoacid substitution is obtained using methods well known in the art. In yet a further preferred embodiment, F, W, T, H or Y in position P1 is deleted. In yet another embodiment, F, W, T, H or Y in positions P1 and P7 are deleted. Methods to carry out said deletions are well known in the art. In yet another particular embodiment at least one aminoacid is added within the CDld binding motif, in any location within the P1 to P7 sequence.
   According to the present disclosure medicaments are envisaged for the treatment of diseases wherein administration of allofactors are required, such as in:
   (1) congenital or acquired deficiency in factors associated with coagulation (such as factor VIII, factor IX or factor X) or fibrinolysis, with defect in enzymes associated with the metabolism of polysaccharides or glycogen (such as in Pompe disease), or with defect in hormone production (such as insulin in diabetes or growth hormone in nanism)
   (2) acute or chronic situations wherein it is advantageous to administer a curative agent, such as thrombolytic agents including staphylokinase and microplasmin,
   (3) disorders of the immune system in which it is required to administer cytokines (or their receptor) or growth factors (such as interferon-alpha, interferon-beta, interferon-gamma, G-CSF, GM-GSF, KGF or erythropoietin)
(4) diseases characterized by chronic inflammation or inappropriate immune responses, wherein therapeutic antibodies should be administered, including anti-tumor necrosing factor, anti-CD3 or anti-CD4 antibodies in autoimmune diseases and graft rejection, antibodies to lymphocyte surface markers (such as anti-CD20 antibodies in non-Hodgkin lymphomas), or antibodies to factor VIII in the prevention of thrombosis. The list of therapeutic antibodies is growing fast and the present invention intends to cover the use of any antibodies used for therapeutic purposes in general.
   According to the present disclosure medicaments are also envisaged for use in gene therapy and gene vaccination, wherein viral vectors are utilized and wherein the immune response against said vectors precludes transgene expression.
   According to the present disclosure medicaments are also envisaged for diseases elicited by exposure to environmental proteins, such as:
   (1) proteins to which said subject is exposed by food or feed. Examples of these are cereals such as wheat, maize, rice, soybean and colza, vegetables such as potato and beetroot, fruits such as rosacea, nuts, and avocado, enzymes, anti-viral or anti-bacterial drugs.
   (2) proteins towards which the subject is exposed by inhalation, systemic route or by stinging. Examples of these are allergic reactions to pollens, contact reaction to latex or hymenoptera stings.
      According to the present disclosure medicaments are also envisaged for immunization (vaccination) such as:
      (1) vaccination against allergens
      (2) vaccination against infectious agents, including viruses, bacteria and parasites

In both these circumstances it may be advantageous to prevent an activation of the innate immune system so as to prevent excess of inflammation and its detrimental consequences on the result of said vaccination. Another advantage in the setting of vaccination to allergens or infectious agents is that the elimination of NKT cell activation prevents the suppressive effect of activated NKT cells on the developemnt of an adaptive response against said allergens or said infectious agents.

It should be recognized that the above list is not exhaustive and that the invention intends to cover newly-introduced products such as antibodies, cytokines, growth factors or peptides and polypeptides used for replacement in congenital or acquired deficiencies, and genetically-modified proteins.

It should be understood that any of the peptides or polypeptides listed above may be administered in the form of gene for transgenesis, which may be carried out using viral vectors or other means known by those skilled in the art. In such a case, the viral vector itself may be modified according to the present invention by eliminating CDld binding motifs.

The medicament is usually, though not necessarily, a (pharmaceutical) formulation comprising as active ingredient at least one of the peptides or polypeptides of the invention or a gene therapeutic vector capable of expressing said peptides or polypeptides. Apart from the active ingredient(s), such formulation will comprise at least one of a (pharmaceutically acceptable) diluent.

A notable exception to this rule is the use of proteins from genetically-modified organisms for food or feed, exposure by inhalation or by the systemic route.

In general, administration of peptides or polypeptides of the invention prevents activation of the innate immune system, more particularly activation of NKT cells, more particularly the production of cytokines associated with NKT cell activation.

The route of administration for peptides or polypeptides of the present invention may vary according to the indication and/or the nature of the peptides or polypeptides. Examples are intravenous injection of coagulation factors, subcutaneous injection of insulin and oral administration of genetically-modified proteins. The present invention intends to cover all other possible routes of administration such as intranasal, sublingual, percutaneous, intramuscular, intrarectal or intravaginal.

As explained in detail further on, the peptides or polypeptides of the present invention can be made by chemical synthesis, which further allows the incorporation of non-natural amino acids. The peptides or polypeptides of the present invention can also be produced using methods know in the art for the production of recombinant proteins using expression systems such as bacterial cells, yeast cells, insect cells, plant cells or mammalian cells.

Another aspect of the present invention relates to methods for generating peptides and polypeptides of the present invention described herein. Such methods include the identification of NKT-cell epitopes from allofactors, viral vectors, proteins to which said subject is exposed by food, feed, systemic or inhalation route, allergens or specific infectious agents. Ways for *in vitro* and *in silico* identification NKT-cell epitopes are amply known in the art and some aspects are elaborated upon hereafter.

The identification of a NKT-cell epitope in the context of the present invention is known to a person skilled in the art. For instance, peptide sequences isolated from allofactors, viral vectors, proteins to which said subject is exposed by food, feed, systemic or inhalation route, genetically-modified proteins, allergens or specific infectious agents are tested by, for example, NKT cell biology techniques, to determine whether the peptide sequences elicit a NKT cell response. Those peptide sequences found to elicit a NKT cell response are defined as having NKT cell stimulating activity. Mammal NKT cell stimulating activity can further be tested by culturing NKT cells obtained from an individual sensitized to an allofactor, viral vector, proteins to which said subject is exposed by food, feed, systemic or inhalation route, genetically-modified protein, allergen or specific infectious agent, and determining whether proliferation of NKT cells occurs in response to the peptide/epitope as measured, e.g., by cellular uptake of tritiated thymidine. Stimulation indices for responses by NKT cells to peptides/epitopes can be calculated as the maximum CPM in response to a peptide/epitope divided by the control CPM. A NKT cell stimulation index (S.I.) equal to or greater than two times the background level is considered "positive." Positive results are used to calculate the mean stimulation index for each peptide/epitope for the group of peptides/epitopes tested. Non-natural NKT-cell epitopes can further optionally be tested for their binding affinity to CDld molecules. The binding of non-natural NKT-cell epitopes to CDld molecules can be performed in different ways. For instance, soluble CDld molecules are obtained and made tetrameric by synthesis and/or chemical coupling. The CDld molecule is purified by affinity chromatography. Soluble CDld molecules are incubated with a biotin-labeled reference peptide produced according to its strong binding affinity for said CDld molecule. Peptides to be assessed for CDld binding are then incubated at different concentrations and their capacity to displace the reference peptide from its CDld binding site is calculated by addition of neutravidin. Methods can be found in for instance in Texier et al., (2000) J. Immunology 164, 3177-3184) for peptides presented by the MHC class II determinants, but the method can easily be applied to CD1d-restricted NKT cell epitopes. The immunogenic peptides or polypeptides of the invention have a mean NKT cell stimulation index of greater than or equal to 2. An immunogenic peptide having a NKT cell stimulation index of greater than or equal to 2 is considered useful as a candidate to carry out the substitution or deletion of hydrophobic aminoacid residues, or addition of aminoacids within the sequence of the CDld binding motif, as described in the present invention.

If two or more aminoacid sequences which share an area of overlap in the native peptide or polypeptide sequence are found to have human NKT cell stimulating activity, as determined by T cell biology techniques, mutation or deletion of hydrophobic aminoacid residues may be carried out for residues belonging to one or to both of the sequences.

The peptides or polypeptides of the invention can be produced by recombinant expression in, e.g., bacterial cells (e.g. *Escherichia coli*), yeast cells (e.g., *Pichia* species, *Hansenula* species, *Saccharomyces* or *Schizosaccharomyces* species), insect cells (e.g. from *Spodoptera frugiperda* or *Trichoplusia ni*), plant cells or mammalian cells (e.g., CHO, COS cells). The construction of the therefore required suitable expression vectors (including further information such as promoter and termination sequences) involves meanwhile standard recombinant DNA techniques. Recombinantly produced peptides or polypeptides of the invention can be derived from a larger precursor protein, e.g., via enzymatic cleavage of enzyme cleavage sites inserted adjacent to the N- and/or C-terminus of the peptide or polypeptide, followed by suitable purification.

In view of the limited length of some of the peptides of the invention, they can be prepared by chemical peptide synthesis, wherein peptides are prepared by coupling the different amino acids to each other. Chemical synthesis is particularly suitable for the inclusion of e.g. D-amino acids, amino acids with non-naturally occurring side chains or natural amino acids with modified side chains such as methylated cysteine. Chemical peptide synthesis methods are well described and peptides can be ordered from companies such as Applied Biosystems and other companies. Peptide synthesis can be performed as either solid phase peptide synthesis (SPPS) or contrary to solution phase peptide synthesis. The best-known SPPS methods are t-Boc and Fmoc solid phase chemistry which is amply known to the skilled person. In addition, peptides can be linked to each other to form longer peptides using a ligation strategy (chemoselective coupling of two unprotected peptide fragments) as originally described by Kent (Schnolzer & Kent (1992) Int. J. Pept. Protein Res. 40, 180-193) and reviewed for example in Tam et al. (2001) Biopolymers 60, 194-205. This provides the potential to achieve protein synthesis which is beyond the scope of SPPS. Many proteins with the size of 100-300 residues have been synthesized successfully by this method.

The physical and chemical properties of a peptide or polypeptide of the invention (e.g. solubility, stability) is examined to determine whether the peptide is/would be suitable for use in therapeutic compositions. Typically this is optimized by adjusting the sequence of the peptide. Optionally, the peptide can be modified after synthesis (chemical modifications e.g. adding/deleting functional groups) using techniques known in the art.

The production of genetically-modified organisms relies on methods well known for those skilled in the art, including cloning, site-directed mutagenesis and growth. Disclosed herein are nucleic acid sequences encoding the peptides or polypeptides of the present invention and methods for their use, e.g., for recombinant expression or in gene therapy. In particular, said nucleic acid sequences are capable of expressing peptides of the invention.

In gene therapy, recombinant nucleic acid molecules encoding the peptides or polypeptides of the present invention can be used as naked DNA or in liposomes or other lipid systems for delivery to target cells. Other methods for the direct transfer of plasmid DNA into cells are well known to those skilled in the art for use in human gene therapy and involve targeting the DNA to receptors on cells by complexing the plasmid DNA to proteins. In its simplest form, gene transfer can be performed by simply injecting minute amounts of DNA into the nucleus of a cell, through a process of microinjection. Once recombinant genes are introduced into a cell, they can be recognized by the cell normal mechanisms for transcription and translation, and a gene product will be expressed. Other methods have also been attempted for introducing DNA into larger numbers of cells. These methods include: transfection, wherein DNA is precipitated with calcium phosphate and taken into cells by pinocytosis; electroporation, wherein cells are exposed to large voltage pulses to introduce holes into the membrane); lipofection/liposome fusion, wherein DNA is packed into lipophilic vesicles which fuse with a target cell; and particle bombardment using DNA bound to small projectiles. Another method for introducing DNA into cells is to couple the DNA to chemically modified proteins. Adenovirus proteins are capable of destabilizing endosomes and enhancing the uptake of DNA into cells. Mixing adenovirus to solutions containing DNA complexes, or the binding of DNA to polylysine covalently attached to adenovirus using protein crosslinking agents substantially improves the uptake and expression of the recombinant gene. Adeno-associated virus vectors may also be used for gene delivery into vascular cells. As used herein, "gene transfer" means the process of introducing a foreign nucleic acid molecule into a cell, which is commonly performed to enable the expression of a particular product encoded by the gene. The said product may include a protein, polypeptide, anti-sense DNA or RNA, or enzymatically active RNA. Gene transfer can be performed in cultured cells or by direct administration into mammals. In another embodiment, a vector comprising a nucleic acid molecule sequence encoding a peptide according to the invention is provided. In particular embodiments, the vector is generated such that the nucleic acid molecule sequence is expressed only in a specific tissue. Methods of achieving tissue-specific gene expression are well known in the art, e.g., by placing the sequence encoding an immunogenic peptide of the invention under control of a promoter, which directs expression of the peptide specifically in one or more tissue(s) or organ(s). Expression vectors derived from viruses such as retroviruses, vaccinia virus, adenovirus, adeno-associated virus, herpes viruses, RNA viruses or bovine papilloma virus, may be used for delivery of nucleotide sequences (e.g., cDNA) encoding peptides, homologues or derivatives thereof according to the invention into the targeted tissues or cell population. Methods which are well known to those skilled in the art can be used to construct recombinant viral vectors containing such coding sequences. Alternatively, engineered cells containing a nucleic acid molecule coding for a peptide or polypeptide according to the invention may be used in gene therapy.

The medicament is usually, but not necessarily (for instance proteins produced by genetically-modified organisms which are used for food, feed, or to which said subject is exposed to systemic or inhalation route), a (pharmaceutical) formulation comprising as active ingredient at least one of the peptides or polypeptides of the invention, a gene therapeutic vector capable of expressing said peptide or polypeptide. Apart from the active ingredient(s), such formulation will comprise at least one of a (pharmaceutically acceptable) diluent. Typically, pharmaceutically acceptable compounds can be found in, e.g., a Pharmacopeia handbook (e.g. US-, European- or International Pharmacopeia). The medicament or pharmaceutical composition of the invention normally comprises a (prophylactically or therapeutically) effective amount of the active ingredient(s) wherein the effectiveness is relative to the condition or disorder to be prevented or treated.

The medicament or pharmaceutical composition may need to be administered to a subject in need as part of a prophylactic or therapeutic regimen comprising multiple administrations of said medicament or composition. Said multiple administrations usual occur sequentially and the time-interval between two administrations can vary and will be adjusted to the nature of the active ingredient and the nature of the condition to be prevented or treated. The amount of active ingredient given to a subject in need of a single administration can also vary and will depend on factors such as the physical status of the subject (as for instance weight and age), the status of the condition to be prevented or treated, and the experience of the treating doctor, physician or nurse.

The term "diluents" refers for instance to physiological saline solutions. The term "pharmaceutically acceptable carrier" means any material or substance with which the active ingredient is formulated in order to facilitate its application or dissemination to the locus to be treated, for instance by dissolving, dispersing or diffusing the said composition, and/or to facilitate its storage, transport or handling without impairing its effectiveness. They include any and all solvents, dispersion media, coatings, antibacterial and antifungal agents (for example phenol, sorbic acid, chlorobutanol), isotonic agents (such as sugars or sodium chloride) and the like. Additional ingredients may be included in order to control the duration of action of the active ingredient in the composition. The pharmaceutically acceptable carrier may be a solid or a liquid or a gas which has been compressed to form a liquid, i.e. the compositions of this invention can suitably be used as concentrates, emulsions, solutions, granulates, dusts, sprays, aerosols, suspensions, ointments, creams, tablets, pellets or powders. Suitable pharmaceutical carriers for use in said pharmaceutical compositions and their formulation are well known to those skilled in the art. They may also include additives such as wetting agents, dispersing agents, stickers, adhesives, emulsifying agents, solvents, coatings, antibacterial and antifungal agents (for example phenol, sorbic acid, chlorobutanol), isotonic agents (such as sugars or sodium chloride) and the like, provided the same are consistent with pharmaceutical practice, i.e. carriers and additives which do not create permanent damage to mammals. The pharmaceutical compositions may be prepared in any known manner, for instance by homogeneously mixing, coating and/or grinding the active ingredients, in a one-step or multi-steps procedure, with the selected carrier material and, where appropriate, the other additives such as surface-active agents. They may also be prepared by micronisation, for instance in view to obtain them in the form of microspheres usually having a diameter of about 1 to 10 µm, namely for the manufacture of microcapsules for controlled or sustained release of the active ingredients.

Peptides or polypeptides, homologues or derivatives thereof (and their physiologically acceptable salts or pharmaceutical compositions all included in the term "active ingredients") may be administered by any route appropriate to the condition to be prevented or treated and appropriate for the compounds, here the peptide or polypeptide to be administered. Possible routes include regional, systemic, oral (solid form or inhalation), rectal, nasal, topical (including ocular, buccal and sublingual), vaginal and parenteral (including subcutaneous, intramuscular, intravenous, intradermal, intraarterial, intrathecal and epidural). The preferred route of administration may vary with for example the condition of the recipient or with the condition to be prevented or treated.

The formulations may conveniently be presented in unit dosage form and may be prepared by any of the methods well known in the art of pharmacy. Formulations suitable for oral administration may be presented as discrete units such as capsules, cachets or tablets each containing a predetermined amount of the active ingredient; as a powder or granules; as solution or a suspension in an aqueous liquid or a non-aqueous liquid; or as an oil-in-water liquid emulsion or a water-in-oil liquid emulsion. The active ingredient may also be presented as a bolus, electuary or paste. A tablet may be made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Viral vectors for the purpose of gene therapy or gene vaccination are highly amenable to modifications by means of recombinant nucleic acid technology. In view of the above, a skilled person will further easily envisage that the elimination of the viral vector NKT-cell epitope as applied in the peptides or polypeptides and their uses can be introduced immediately in the viral vector itself. Hence, the disclosure further encompasses modified viral vectors defined as isolated viral vectors characterized in that CDld binding motifs have been eliminated by aminoacid substitution or deletion.

The present invention will now be illustrated by means of the following examples, which are provided without any limiting intention. Furthermore, all references described herein are explicitly included herein by reference.

### Examples

### Example 1: Coagulation factor VIII

Patients suffering from hemophilia A lack sufficient amounts of factor VIII (FVIII), which is the reason for uncontrolled bleeding tendency. Such patients are treated by infusions of FVIII purified from plasma source or produced by recombinant technology. Administration of FVIII results in the formation of specific antibodies, which in more or less 30% of the cases inhibit the function of FVIII as a coagulation cofactor.

Using an algorithm, we identified within the sequence of the FVIII molecule 3 sequences bearing a CDld binding sequence, which matched the [FWTHY]- X₂X₃-[ELMV]-X₅X₆-[FWTHY] sequence motif. These motifs are located in the A1 and A3 domains, respectively:
FCHISSH in A1 domain (aminoacids 309-315, SEQ ID1)
FWKVQHH in A3 domain (aminoacids 1816-1822, SEQ ID2)
FHAINGY in A3 domain (aminoacids 1918-1924, SEQ ID3)

These sequences have in common (underlined) an aromatic residue (phenylalanine, F) in position 1, an aliphatic residue (isoleucine, I, or valine, V) in position 4, and an aromatic residue (histidine, H, or tyrosine, Y) in position 7.

To determine whether these sequences could activate NKT cells in vivo, FVIII (2 IU) was injected intravenously to hemophilia A mice on 4 occasions separated by a 1-week interval. Hemophilia A mice produce no FVIII due to a stop codon introduced in the FVIII gene in exon 16.

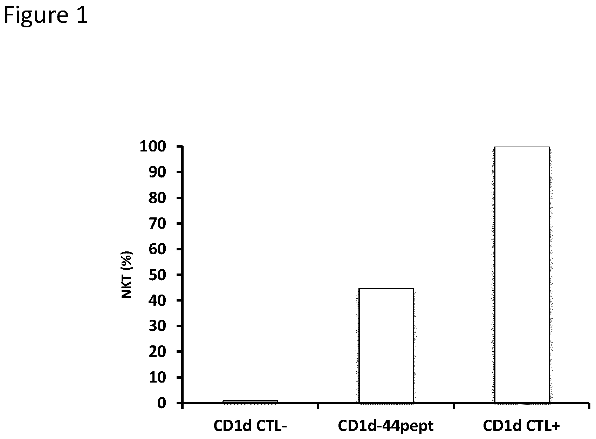

Mice were sacrificed 10 days after the last injection, the spleen was removed and CD4+ T cells were prepared by magnetic bead sorting. NKT cells are characterized by expression of CD4 and recognition of antigen presented by CDld molecule. A tetramer of CDld was obtained from a commercial supplier and loaded with 15 aminoacid long FVIII peptides, which included peptides containing SEQ ID1, SEQ ID2 and SEQ ID3. Significant binding of CDld tetramers loaded with these peptides was observed, indicating that these 3 peptides were able to bind to CDld and that injection of FVIII elicited activation of NKT cells. Representative results are given in Figure 1.

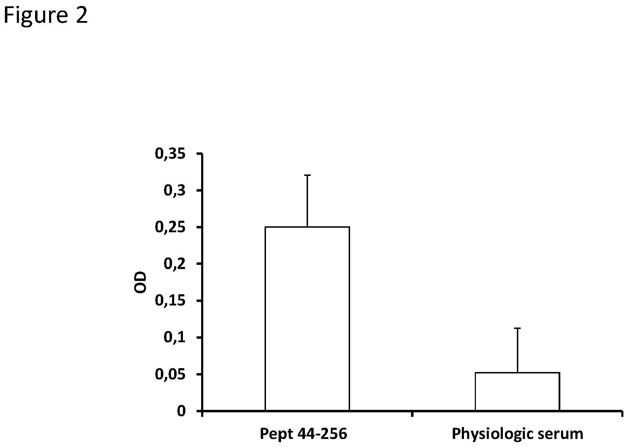

Further, direct immunization with peptides containing a CDld motif (peptides of SEQ ID1, SEQ ID2 or SEQ ID3) was sufficient as to elicit the activation of NKT cells and production of antibodies to FVIII. Prominent activation was observed with peptide of SEQ ID1. Representative results are given in Figure 2.

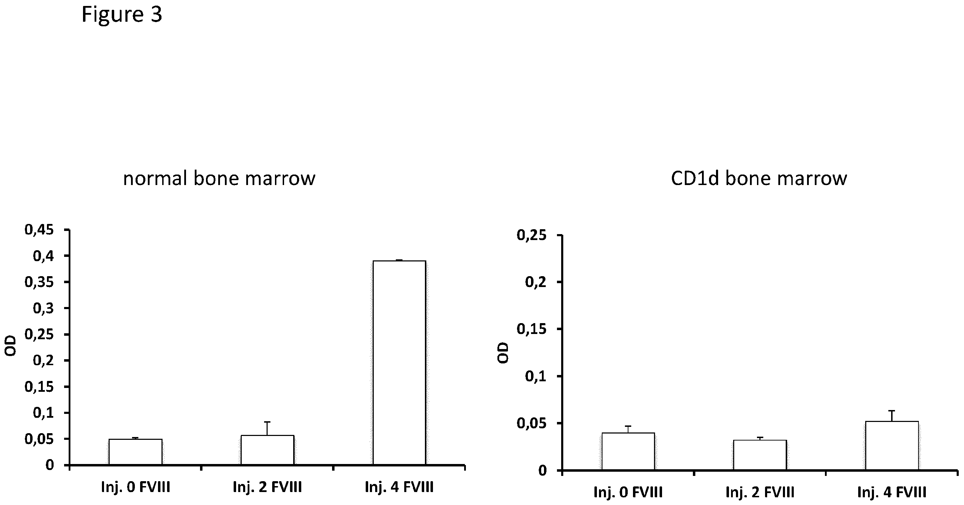

Bone marrow chimeras were constructed in which hemophilia A mice were first irradiated and reconstituted with the bone marrow of mice lacking NKT cells, namely CDld knocked-out mice. In the absence of NKT cells, mice were unable to produce significant amounts of antibodies to FVIII, and virtually no antibodies inhibiting the function of FVIII (Figure 3).

The A1 domain of FVIII was produced by recombinant technology in its natural sequence or with a substitution of F309 and H315 by serine (polypeptide of SEQ ID4). FVIII A1 domains in natural sequence or SEQ ID4 were used to immunize separate groups of mice. The results showed that substitution of F309 and H315 by S (SEQ ID4) was sufficient to prevent activation of NKT cells as assessed from spleen CD4+ T cells as described above.

The A3 domain of FVIII was produced by recombinant technology in its natural sequence or with a substitution of F1816 and H1822 by serine (polypeptide of SEQ ID 5). FVIII A3 domains in natural sequence or SEQ ID5 were used to immunize separate groups of mice. The results showed that substitution of F1816 and H1822 by S (SEQ ID5) was sufficient to prevent activation of NKT cells as assessed from spleen CD4+ T cells as described above.

A B domain-deleted FVIII molecule in its natural sequence elicited activation of NKT cells (see above). A FVIII molecule with 4 aminoacid substitutions was prepared containing F309S, H315S, F1816S and F1918S (SEQ ID6). Intravenous injections of such mutated FVIII in hemophilia A mice did not result in the formation of antibodies to FVIII and, consequently, no antibodies inhibiting the function of FVIII.

It was therefore concluded that:
(1) FVIII naturally contains several CDld binding motifs;
(2) these CDld motifs are functional and elicit activation of NKT cells;
(3) activation of NKT cells is a requisite for the production of antibodies to FVIII;
(4) elimination of the CDld motifs by aminoacid substitution is sufficient to eliminate the production of anti-FVIII antibodies

It should be clear for those skilled in the art that the invention also applies to animals made transgenic for the production of coagulation factors such as factor IX.

### Detailed description of the drawings

### Figure 1

### NKT cells recognize factor VIII epitopes presented by CDld

Hemophilia A mice were immunized with 2 IU Factor VIII on 4 occasions separated by one week. The spleen was then removed and CD4+ T cells were prepared by magnetic bead sorting. A fluorochrome-labeled tetramer of CDld was obtained from a commercial supplier and loaded with 15 aminoacid long FVIII peptides, which included peptides containing SEQ ID1, ID2 and ID3. Loading was carried out at room temperature overnight in the dark. Tetramers were then incubated for 30 minutes at 4°C with the CD4+ T cell population and the cell suspension was analyzed by Facs.

The figure shows that CD1d tetramers loaded with peptide of SEQ ID1 (44pept in the figure) are recognized by NKT cells. CDld ctl(-) shows the % of NKT cells recognizing unloaded tetramers. CDld ctl(+) shows the % of NKT cells recognizing tetramers loaded with alpha-gal ceramide, which recruits all NKT cells. Up to 45% of NKT cells recognize 44pept, which is compatible with the absence of polymorphism at CDld level and very limited polymorphism at the level of the NKT T cell receptor.

### Figure 2

### Immunization of hemophilia A mice with a CD1d-restricted peptides elicits anti-factor VIII antibodies

Hemophilia A mice were immunized 3 times subcutaneously with 50 µg of an equimolar mixture of peptides of SEQ ID1 (44pept) and peptide of SEQ ID2 (256pept) adsorbed on aluminum hydroxyde. A control group received physiological serum instead of peptides. Plasma was taken 10 days after the last immunization and assessed for the presence of anti-Factor VIII antibodies, using a direct binding assay. Briefly, Factor VIII (10 IU/ml) was insolubilized on polystyrene plates, which were washed and incubated with a 1/10 dilution of plasma. After a further washing, a HRP-labeled goat anti-mouse antiserum was added, followed by an enzyme substrate. Colour development was read as OD.

The figure shows that hemophilia A mice immunized with peptides of SEQ ID1 and of SEQ ID2 develop antibodies to Factor VIII.

### Figure 3

### Hemophilia A mice reconstituted with bone marrow from CDld KO mice do not produce antibodies to Factor VIII

Hemophilia A mice were lethally irradiated and reconstituted with the bone marrow of CDld KO mice (5x10⁶/mouse), which lack NKT cells. Six weeks after bone marrow reconstitution the mice received 4 IV injections of 2 IU/ml separated by one week. Mice were bled 10 days after the last immunization and the plasma was assayed for the presence of anti-Factor VIII antibodies using a direct binding assay as described in the legend of Figure 2. A control group of irradiated hemophilia A mice was reconstituted with a normal bone marrow.

The figure indicates that, although control mice produce high concentrations of anti-FVIII antibodies after the fourth injection of Factor VIII (left panel), mice reconstituted with the bone marrow of NKT cell deficient mice did not (right panel).

### Example 2: adenovirus 5 viral vectors

Viral vectors are commonly used for gene therapy and gene vaccination. One of the most common of these viral vectors is derived from adenovirus, serotype 5. Adenoviruses (Ad) are non-enveloped viruses possessing a linear, double-stranded DNA genome of about 35 kb. Human Ad5 has a capsid consisting of 3 major structural proteins: hexon, penton, and fiber. Neutralizing antibodies are raised towards hexon proteins. Such antibodies are very common in humans as a consequence of viral infection. The presence of such antibodies blocks the entry of the viral vector and, consequently, prevents expression of the transgene protein carried by the vector. Anti-Ad5 antibodies are generated in the course of an adaptive response, which depends on activation of CD4+ T cells specific for epitopes presented in the context of MHC class II molecules.

It is known that Ad5 activates the innate immune system, though the precise mechanism by which it occurs and the location where it takes place remain unclear. Yet, activation of the innate immune system could be a required step for neutralizing antibodies to be formed.

Using algorithms, we identified 7 aminoacid sequences matching with the general motif [FWTHY]- X₂X₃-[ILMV]-X₅X₆-[FWTHY] of a CDld binding sequence (SEQ ID7, with motifs underlined) in hexon 6.

Mice were injected intravenously with 10⁹ PFU Ad5 vector on 3 occasions at 10-day intervals. CD4+ T cells were then prepared from the spleen by magnetic bead sorting. CD4+ T cells were incubated with CDld tetramers loaded with peptides corresponding to each of the 7 sequences identified. It showed that a significant proportion (± 10%) of CD4+ NKT cells were labeled by tetramers, indicating that Ad5 vector injections activated NKT cells specific for the peptide of SEQ ID7. In addition, such mice produced specific antibodies of the IgG2a isotype, characteristic of neutralizing antibodies in the mouse.

A viral vector was prepared which contained a substitution of [FW] by serine S for each of the 7 aminoacid sequences identified. This mutated viral vector (SEQ ID8, with underlined motifs) was used to immunize animals according to the same protocol as described above for the natural sequence. The proportion of NKT cells as assessed using tetramers loaded with the peptide in natural sequence (SEQ ID7) was <1% and the concentration of Ad5 virus specific antibodies was significantly reduced (up to 10-fold).

It was therefore concluded that substitution of F to S in each P1 location of CDld binding motifs was sufficient as to reduce NKT cell activation and thereby reduce the production of anti-Ad5 antibodies.

### Example 3: genetically-modified proteins

Proteins to which subjects are exposed by way of inhalation or ingestion are frequently eliciting unwanted reactions in predisposed subjects. Allergic asthma affects millions of people across the world. Food allergy on the other hand has an overall prevalence of ± 2.5% in the general population. Allergens either airborne, ingested or penetrating the skin could share properties by which they activate NKT cells.

One of the most common food allergen is apple (Malus domesticus), and allergenicity is almost exclusively borne by the Mal d 1 protein, a 159 aminoacid long protein, which protects the plant against infectious agents. A sequence motif was identified using computer algorithms, which corresponds to the general motif [FWTHY]- X₂X₃-[ILMV]-X₅X₆-[FWTHY] of a CD1d binding sequence.

FKLIESY corresponding to aminoacids 144-150 of Mal d 1 (SEQ ID9)

A recombinant form of Mal d 1, in which F144 and Y150 were mutated in S was produced by genetic engineering. The recombinant form of Mal d 1 therefore encompasses peptide of sequence:
SKLIESS (SEQ ID10)

Synthetic peptides corresponding to SEQ ID9 and SEQ ID10 were produced. Their capacity to activate NKT cells was determined in vitro using human dendritic cells derived from peripheral blood monocytes of an individual sensitized to Mal d 1. Dendritic cells loaded with each one of the two peptides were incubated in the presence of NKT cells obtained from the same individual by sorting peripheral lymphocytes using specific markers such as CD4 and NKG2D. It was observed that NKT cells incubated with peptide of SEQ ID9 activated a significant proportion of NKT cells, while the mutated peptide of SEQ ID10 did not. Additionally, human CD1d tetramers loaded with peptides of SEQ ID9 were recognized by a significant proportion of NKT cells, but tetramers loaded with the mutated peptide of SEQ ID10 were recognized by less than 1% of NKT cells.

The two F144S and Y150S mutations are introduced directly in clonal cells by site-directed mutagenesis. The full organism is then produced by conventional growth strategies. Apples produced by this GMO do not elicit allergic reactions.

One specific application of the peptides or polypeptides of the present invention is celiac disease (gluten intolerance). This disease is among the most commons in human beings and is related to T cell activation to gliadin epitopes which are presented in the context of MHC class II determinants. A genetic susceptibility has been described, with human beings carrying the HLA-DQ2 or DQ8 class II determinant being predisposed to disease. These class II determinants present peptides which have been submitted to deamidation by transglutaminase. However, these events are the results of intestinal inflammatory reaction, likely related to the innate immune system.

Gliadins are monomers of 250-300 aminoacid residues. A search for the general motif [FW]-XX-[ILM]-XX-[FWTHY] of a CDld binding sequence using computer algorithms identified such sequence (SEQ ID11, see listing of sequences) in alpha-gliadin. A mutated form of alpha-gliadin was then produced in which the F residue of the motif was substituted by a S residue (SEQ ID12, see addendum).

The same procedure as for Mal d 1 was followed to show that, although polypeptide of SEQ ID 11 activated a significant proportion of NKT cells when presented by antigen-presenting dendritic cells, the mutated form of the polypeptide (SEQ ID 12) failed to do so. As for Mal d 1, human CD1d tetramers loaded with a synthetic peptide representing the motif identified in the polypeptide of

SEQ ID11 were recognized by NKT cells, while tetramers loaded with the mutated form of the motif as shown in SEQ ID 12 were not.

The mutation was introduced directly in clonal cells by site-directed mutagenesis. The full organism was then produced by conventional growth strategies. Cereals containing the mutated form of gliadin do not elicit reactions of intolerance.

It should be obvious for those skilled in the art that the present invention can also be applied to proteins which are added to, for instance, genetically-modified organisms to increase their resistance to insecticides, pesticides or any other modifications judged to be beneficial. Such modifications carry the risk of creating new CD1d binding motifs.

Additional examples of genetically-modified proteins with reduced allergenicity/immunogenicity are:
- food allergens such as soybean, peanut and fruits of the Rosaceous family
- milk proteins
- airborne allergens such as latex (Hevea brasiliensis), pollens of grasses such as Rye grass (Lolium perenne), Timothy (Phleum pratense) or Kentucky blue grass (Poa pratensis)
- fish parvalbumin
- honey bee phospholipase A2

It should also be clear for the one skilled in the art that the invention extends to methods by which peptides or polypeptides of the invention are produced, including the production of transgenic plants and animals.

### Example 4: allergen Der p 1

Der p 1 is a cysteine protease which is the main allergen of the so-called house dust mite (HDM), D. pteronyssinus. Sensitization to HDM is by far the commonest trigger of allergic asthma and rhinitis worldwide. Der p 1 contains 3 motifs matching the general CDld binding motif [FWTHY]- X₂X₃-[ILMV]-X₅X₆-[FWTHY], as identified using computer algorithms and which are:
SEQ ID13: FSGVAAT aminoacids 38-44 of Der p 1
SEQ ID14: HSAIAAVI aminoacids 135-141 of Der p 1
SEQ ID15: YPYVVIL aminoacids 216-222 of Der p 1

Peptides of SEQ ID 13, SEQ ID14 and SEQ ID 15 were synthesized and used to load CD1d tetramers.

BALB/c mice were submitted to intranasal administration of Der p 1, using 50 µl of saline containing 100 µg of Der p 1. This challenge procedure was repeated twice on three consecutive days at one-week interval. The mice were sacrificed 5 days after the last nasal instillation and the spleen was removed. CD4+ T cells were purified by magnetic bead sorting and incubated in the presence of the CD1d tetramers loaded with peptides of SEQ ID 13, SEQ ID 14 or SEQ ID 15. By fluorescence-activated cell sorter (facs) determination, it was observed that a significant percentage of cells (± 10%) were stained with the tetramers, identifying them as CD4+ NKT cells. It was therefore concluded that peptides of SEQ ID13, SEQ ID14 and SEQ ID 15 were functional in binding to CD1d and in being recognized by NKT cells.

CD4+ T cells obtained from the above experiments were incubated in culture medium in the presence of an antigen-presenting cell which expresses the CDld molecule. Such cells are commercially available, as for instance the JAWS2 cells, which do not express MHC class II determinants. JAWS2 cells were loaded with Der p 1 and presentation of Der p 1-derived epitopes by CDld was evaluated by measuring the production of cytokines such as IFN-gamma and IL-4 as markers of NKT activation. It could be observed that a significant production of cytokines was present, confirming that Der p 1 contained epitopes presented by CD1d molecules.

Next, a mutated form of Der p 1 was prepared by genetic engineering, in which the 3 aminoacid residues predicted to be in position P1 for CDld binding of peptides of SEQ ID13, SEQ ID14 and SEQ ID15 were substituted by serine. The mutated Der p 1 (SEQ ID16) was used for nasal instillation as described above with Der p 1 in natural sequence (SEQ ID 17). In such a case, no significant binding of CD4+ T cell splenocytes was observed when incubated with the tetramers loaded with peptide of SEQ ID13, peptide of SEQ ID14 or peptide of SEQ ID 15, indicating that the mutated Der p 1 had lost its capacity to activate NKT cells specific for these peptides.

Further, mutated Der p 1 (SEQ ID16) was used to load JAWS2 cells and tested for its capacity to activate NKT cells. For this experiment, NKT cells were used as obtained from mice immunized with Der p 1 in either natural or mutated configuration. The production of IFN-gamma and IL-4 was taken as an indication of NKT activation. It was observed that NKT cells obtained from mice immunized with natural sequence Der p 1 failed to be activated when incubated in the presence of JAWS2 cells loaded with mutated Der p 1.

It was therefore concluded that Der p 1 in natural sequence contained functional CDld restricted T cell epitopes activating NKT cells. Further, elimination of such functional CD1d-restricted epitopes by mutation was sufficient to eliminate NKT cell activation.

### Example 5: antibodies

Antibodies are used as therapeutic agents in a large number of indications, from chronic inflammatory diseases such as rheumatoid arthritis (e.g., anti-TNF-alpha antibodies) or allergic asthma (e.g. anti-IgE antibodies), to tumors (e.g., anti-CD20 antibodies). More than 120 therapeutic antibodies are presently used for clinical applications at various stages from preclinical to phase III trials and accepted for routine clinical practice.

Therapeutic antibodies are either chimeric or fully humanized, which contains sequence of foreign origin only in the complementarity determining regions of the variable parts. A minority of such antibodies are derived from the human repertoire and, as such, considered as poorly immunogenic. However, antibodies towards the therapeutic antibody, even when directly derived from the human repertoire, are produced by a majority of the patients under treatment, with, in a significant proportion of the cases, the production of antibodies neutralizing the activity of the therapeutic agent.

A search for epitopes matching the CD1d binding motif in human IgG antibody sequence was carried out using computer algorithms. One of such motif was identified in the CH2 region (second domain of the heavy chain constant part) of each of the 4 IgG subclass (IgG1, IgG2, IgG3 and IgG4) and a second motif was identified in the CH3 loop of IgG1, IgG2 and IgG4:
SEQ ID18: YRVVSVL (CH2 of IgG1 and IgG4)
SEQ ID19: FRVVSVL (CH2 of IgG2 and IgG3)
SEQ ID20: HEALHNH (CH3 loop of IgG1, IgG2 and IgG4)

Synthetic peptides corresponding to SEQ ID18, SEQ ID 19 and SEQ ID20 were produced and used to load human CD1d tetramers as for the examples above (see for instance example 4 for allergen Der p 1). Peripheral blood cells were obtained by venous puncture of patients who had received an injection of a therapeutic antibody during the previous 5 days. CD4+ T cells were purified by magnetic bead sorting. The cells were then incubated with tetramers loaded with peptides of SEQ ID18, SEQ ID19 or SEQ ID20. Analysis by facs identifies a significant proportion of NKT cells (± 10%) labeled by tetramers.

Monoclonal human antibodies of the IgG4 isotype were derived from the peripheral blood B lymphocytes by transformation with the Epstein-Barr virus. The genomic sequence of such antibodies was obtained from transformed B cells. A viral vector containing the corresponding cDNA sequence was constructed and used for transfection of CHO cells. All these methods are known in the art (see for instance, Jacquemin et al Blood 92: 496-506, 1998).

The hydrophobic aminoacid residues located in position 1 in the peptides of SEQ ID18 and SEQ ID20 were mutated to a serine and the mutated antibody produced by transfected CHO cells.

Peripheral blood CD4+ T cells obtained as above were exposed in culture medium to human dendritic cells (derived from human peripheral blood monocytes by methods known in the art) and loaded with either the antibody in natural configuration (SEQ ID21) or its mutated counterpart (SEQ ID22). After culturing the cells with CD4+ T cells for 5 to 7 days, the population of CD4+ T cells activated by either natural or mutated antibody was evaluated. CD4+ NKT cells were separated from CD4+ T cells using an antibody to NKG2D, a surface marker associated with NK or NKT cells only.

It was observed that CD4+ T cells and NKT cells were activated when the antibody in natural sequence was used (SEQ ID21), while the mutated form of the antibody (SEQ ID22) only activate class II restricted CD4+ T cells and not NKT cells.

It was concluded that human IgG antibodies contained epitopes corresponding to the [FWTHY]- X₂X₃-[ILMV]-X₅X₆-[FWTHY] motif, having the capacity to be recognized by and to activate NKT cells. Further, mutation of key hydrophobic aminoacid residues within such motif was sufficient to prevent activation of NKT cells.

It should be understood that the examples provided here are not exhaustive and that combinations of proteins or peptides containing various numbers of aminoacid substitutions or deletions can be envisioned. For instance, in example 1, various combinations of substitution of hydrophobic aminoacids can be delineated.

### Sequence listings

**SEQ ID1**
   Factor VIII aminoacids 309-315 (human)
   FCHISSH
**SEQ ID2**
   Factor VIII aminoacids 1816-1822 (human)
   FWKVQHH
**SEQ ID3**
   Factor VIII aminoacids 1918-1924 (human)
   FHAINGY
**SEQ ID4**
   Factor A1 domain (mutations F309S and H315S underlined) (human) 
1 ATRRYY LGAVELSWDYMQSDLGELP VDAR FPP RV P K S FPF

41 NTS VVYKKT LFVE F T VHLFNI AKPR P PWMGLLGPTI QA EV

81 YDT VVITLKNMASHPVSLHAVGVS Y W K ASEGAEYDDQTSQ
 
121 REK EDDK VFPGGSHTYVWQVLKE N G P MASDPLCLTYSYLS

161 HVD LVK DLNSGLIG AL LVCRE GSLA K E KTQ TL HKFILLFA

201 VFD EGKSWHSE TKN SLMQDRDAASARAWPKMHTVNGYVNR

241 SLP GLIGCH R KSV YWH VIG MGTT PEV HSIF LEG HTFL VRN

281 HRQ AS LEI SPIT FLT AQTLLM DL GQFL LSC HIS SS QH DGM

321 EAY VKV DS CPEEP QLRMKNNE EAED YDDDLTDSEMDVVRF

361 D DDN SPSFI Q IRSVA KK HPKTW VHYIA AEEEDW DYAP LVL

401 APDDR SYKS QYLN NGPQ RIGRK YKK VRF MAYT DETFKTRE

441 AIQ H ES G ILGPLLYGE VG D TLL II FK NQ AS RP YNI YP H GI

481 TD VR PLYSR R LPK GVKHLK DFP ILP GEIFK YK WTV TVEDG

521 PTKSDPRCLTRYYSSFVNMERDLASG
**SEQ ID5**
   Factor VIII A3 domain (mutations F1816S and H1822S underlined) (human) 
1637 SQNPPVLKRHQREITRTTLQSDQEEIDYDDTISVEMKKED

1677 FDIYDEDENQSPRSFQKKTRHYFIAAVERLWDYGMSSSPH

1717 VLRNRAQSGSVPQFKKVVFQEFTDGSFTQPLYRGELNEHL

1757 GLLGPYIRAEVEDNIMVTFRNQASRPYSFYSSLISYEEDQR
 
1798 QGAEPRKNFVKPNETKTYSWKVQHSMAPTKDEFDCKAW

1836 AYFSDVDLEKDVHSGLIGPLLVCHTNTLNPAHGRQVTVQE

1876 FALFFTIFDETKSWYFTENMERNCRAPCNIQMEDPTFKEN

1916 YRFHAINGYIMDTLPGLVMAQDQRIRWYLLSMGSNENIHS

1956 IHFSGHVFTVRKKEEYKMALYNLYPGVFETVEMLPSKAGI

1996 WRVECLIGEHLHAGMSTLFLVYSNKCQTPLGMASGHIRDF

2036 QITASGQYGQWAPKLARLHYSGSINAWSTKEPFSWIKVDL

2076 LAPMIIHGIKTQGARQKFSSLYISQFIIMYSLDGKKWQTY

2116 RGNSTGTLMVFFGNVDSSGIKHNIFNPPIIARYIRLHPTH

2156 YSIRSTLRMELMGCDLNSCSMPLGMESKAISDAQITASSY

2196 FTNMFATWSPSKARLHLQGRSNAWRPQVNNPKEWLQVDFQ

2236 KTMKVTGVTTQGVKSLLTSMYVKEFLISSSQDGHQWTLFF

2276 QNGKVKVFQGNQDSFTPVVNSLDPPLLTRYLRIHPQSWVH

2316 QIALRMEVLGCEAQDLY*
**SEQ ID6**
   Factor VIII (mutations F309S, H315S, F1816S and F1918S underlined) (human) ATRRYYLGAVELSWDYMQSDLGELPVDARFPPRVPKSFPFNTSVVYKKTL 
FVEFTVHLFNIAKPRPPWMGLLGPTIQAEVYDTVVITLKNMASHPVSLHA

VGVSYWKASEGAEYDDQTSQREKEDDKVFPGGSHTYVWQVLKENGPMASD

PLCLTYSYLSHVDLVKDLNSGLIGALLVCREGSLAKEKTQTLHKFILLFA

VFDEGKSWHSETKNSLMQDRDAASARAWPKMHTVNGYVNRSLPGLIGCHR

KSVYWHVIGMGTTPEVHSIFLEGHTFLVRNHRQASLEISPITFLTAQTLL

MDLGQFLLSCHISSSQHDGMEAYVKVDSCPEEPQLRMKNNEEAEDYDDDL

TDSEMDVVRFDDDNSPSFIQIRSVAKKHPKTWVHYIAAEEEDWDYAPLVL

APDDRSYKSQYLNNGPQRIGRKYKKVRFMAYTDETFKTREAIQHESGILG

PLLYGEVGDTLLIIFKNQASRPYNIYPHGITDVRPLYSRRLPKGVKHLKD

FPILPGEIFKYKWTVTVEDGPTKSDPRCLTRYYSSFVNMERDLASGLIGP

LLICYKESVDQRGNQIMSDKRNVILFSVFDENRSWYLTENIQRFLPNPAG

VQLEDPEFQASNIMHSINGYVFDSLQLSVCLHEVAYWYILSIGAQTDFLS

VFFSGYTFKHKMVYEDTLTLFPFSGETVFMSMENPGLWILGCHNSDFRNR

GMTALLKVSSCDKNTGDYYEDSYEDISAYLLSKNNAIEPRSFSQNSRHPS

TRQKQFNATTIPENDIEKTDPWFAHRTPMPKIQNVSSSDLLMLLRQSPTP

HGLSLSDLQEAKYETFSDDPSPGAIDSNNSLSEMTHFRPQLHHSGDMVFT

PESGLQLRLNEKLGTTAATELKKLDFKVSSTSNNLISTIPSDNLAAGTDN
 
TSSLGPPSMPVHYDSQLDTTLFGKKSSPLTESGGPLSLSEENNDSKLLES

GLMNSQESSWGKNVSSTESGRLFKGKRAHGPALLTKDNALFKVSISLLKT

NKTSNNSATNRKTHIDGPSLLIENSPSVWQNILESDTEFKKVTPLIHDRM

LMDKNATALRLNHMSNKTTSSKNMEMVQQKKEGPIPPDAQNPDMSFFKML

FLPESARWIQRTHGKNSLNSGQGPSPKQLVSLGPEKSVEGQNFLSEKNKV

VVGKGEFTKDVGLKEMVFPSSRNLFLTNLDNLHENNTHNQEKKIQEEIEK

KETLIQENVVLPQIHTVTGTKNFMKNLFLLSTRQNVEGSYEGAYAPVLQD

FRSLNDSTNRTKKHTAHFSKKGEEENLEGLGNQTKQIVEKYACTTRISPN

TSQQNFVTQRSKRALKQFRLPLEETELEKRIIVDDTSTQWSKNMKHLTPS

TLTQIDYNEKEKGAITQSPLSDCLTRSHSIPQANRSPLPIAKVSSFPSIR

PIYLTRVLFQDNSSHLPAASYRKKDSGVQESSHFLQGAKKNNLSLAILTL

EMTGDQREVGSLGTSATNSVTYKKVENTVLPKPDLPKTSGKVELLPKVHI

YQKDLFPTETSNGSPGHLDLVEGSLLQGTEGAIKWNEANRPGKVPFLRVA

TESSAKTPSKLLDPLAWDNHYGTQIPKEEWKSQEKSPEKTAFKKKDTILS

LNACESNHAIAAINEGQNKPEIEVTWAKQGRTERLCSQNPPVLKRHQREI

TRTTLQSDQEEIDYDDTISVEMKKEDFDIYDEDENQSPRSFQKKTRHYFI

AAVERLWDYGMSSSPHVLRNRAQSGSVPQFKKVVFQEFTDGSFTQPLYRG
 
ELNEHLGLLGPYIRAEVEDNIMVTFRNQASRPYSFYSSLISYEEDQRQGA

EPRKNFVKPNETKTYSWKVQHHMAPTKDEFDCKAWAYFSDVDLEKDVHSG

LIGPLLVCHTNTLNPAHGRQVTVQEFALFFTIFDETKSWYFTENMERNCR

APCNIQMEDPTFKENYRSHAINGYIMDTLPGLVMAQDQRIRWYLLSMGSN

ENIHSIHFSGHVFTVRKKEEYKMALYNLYPGVFETVEMLPSKAGIWRVEC

LIGEHLHAGMSTLFLVYSNKCQTPLGMASGHIRDFQITASGQYGQWAPKL

ARLHYSGSINAWSTKEPFSWIKVDLLAPMIIHGIKTQGARQKFSSLYISQ

FIIMYSLDGKKWQTYRGNSTGTLMVFFGNVDSSGIKHNIFNPPIIARYIR

LHPTHYSIRSTLRMELMGCDLNSCSMPLGMESKAISDAQITASSYFTNMF

ATWSPSKARLHLQGRSNAWRPQVNNPKEWLQVDFQKTMKVTGVTTQGVKS

LLTSMYVKEFLISSSQDGHQWTLFFQNGKVKVFQGNQDSFTPVVNSLDPP

LLTRYLRIHPQSWVHQIALRMEVLGCEAQDLY*GWPLQHLPLPSPLPPQL

QGSVPPWLAFYLCAKS*QTLP*SLL
**SEQ ID7**
   Hexon, Human adenovirus 5, (CD1d binding motifs underlined): (virus) 
MATPSMMPQWSYMHISGQDASEYLSPGLVQFARATETYFSLNNKFRNPTVAPTHDV
TTDRSQRLTLRFIPVDREDTA
YSYKARFTLAVGDNRVLDMASTSFDIRGVLDRGPTFKPYSGTAYNALAPKGAPNPCE
WDEAATALEINLEEEDDDNE
 
DEVDEQAEQQKTHVFGQAPYSGINITKEGIQIGVEGQTPKYADKTFQPEPQIGESQWY
ETEINHAAGRVLKKTTPMK
PCYGSYAKPTNENGGQGILVKQQNGKLESQVEMQFFSTTEAAAGNGDNLTPKVVLY
SEDVDIETPDTHISYMPTIKE
GNSRELMGQQSMPNRPNYIAFRDNFIGLMYYNSTGNMGVLAGQASQLNAVVDLQD
RNTELSYQLLLDSIGDRTRYFS
MWKQAVDSYDPDVRIIENHGTEDELPNYCFPLGGVINTETLTKVKPKTGQENGWEK
DATEFSDKNEIRVGNNFAMEI
NLNANLWRNFLYSNIALYLPDKLKYSPSNVKISDNPNTYDYMNKRVVAPGLVDCYI
NLGARWSLDYMDNVNPFNHHR
NAGLRYRSMLLGNGRYVPFHIQVPQKFFAIKNLLLLPGSYTYEWNFRKDVNMVLQSS
LGNDLRVDGASIKFDSICLY
ATFFPMAHNTASTLEAMLRNDTNDQSFNDYLSAANMLYPIPANATNVPISIPSRNWA
AFRGWAFTRLKTKETPSLGS
GYDPYYTYSGSIPYLDGTFYLNHTFKKVAITFDSSVSWPGNDRLLTPNEFEIKRSVDG
EGYNVAQCNMTKDWFLVQM
LANYNIGYQGFYIPESYKDRMYSFFRNFQPMSRQVVDDTKYKDYQQVGILHQHNNS
GFVGYLAPTMREGQAYPANFP
YPLIGKTAVDSITQKKFLCDRTLWRIPFSSNFMSMGALTDLGQNLLYANSAHALDMT
FEVDPMDEPTLLYVLFEVFD
VVRVHRPHRGVIETVYLRTPFSAGNATT
**SEQ ID8**
   Hexon, Human adenovirus 5 (mutations of P1 anchoring residue underlined): (virus) 
MATPSMMPQWSYMHISGQDASEYLSPGLVQFARATETSFSLNNKFRNPTVAPTHDV
TTDRSQRLTLRFIPVDREDTA
YSYKARFTLAVGDNRVLDMASTSFDIRGVLDRGPTFKPYSGTASNALAPKGAPNPCE
WDEAATALEINLEEEDDDNE
DEVDEQAEQQKTHVFGQAPSSGINITKEGIQIGVEGQTPKYADKTFQPEPQIGESQWY
ETEINHAAGRVLKKTTPMK
PCYGSYAKPTNENGGQGILVKQQNGKLESQVEMQFFSTTEAAAGNGDNLTPKVVLY
SEDVDIETPDTHISYMPTIKE
 
GNSRELMGQQSMPNRPNYIAFRDNSIGLMYYNSTGNMGVLAGQASQLNAVVDLQD
RNTELSYQLLLDSIGDRTRYFS
MWKQAVDSYDPDVRIIENHGTEDELPNYCFPLGGVINTETLTKVKPKTGQENGWEK
DATEFSDKNEIRVGNNFAMEI
NLNANLWRNFLSSNIALYLPDKLKYSPSNVKISDNPNTYDYMNKRVVAPGLVDCYIN
LGARWSLDYMDNVNPFNHHR
NAGLRSRSMLLGNGRYVPFSIQVPQKSFAIKNLLLLPGSYTYEWNFRKDVNMVLQSS
LGNDLRVDGASIKSDSICLY
ATFFPMAHNTASTLEAMLRNDTNDQSFNDYLSAANMLYPIPANATNVPISIPSRNWA
AFRGWASTRLKTKETPSLGS
GYDPYYTYSGSIPYLDGTFYLNHTSKKVAITFDSSVSWPGNDRLLTPNEFEIKRSVDG
EGYNVAQCNMTKDSFLVQM
LANYNIGYQGFYIPESYKDRMYSFFRNFQPMSRQVVDDTKYKDSQQVGILHQHNNS
GFVGYLAPTMREGQAYPANFP
SPLIGKTAVDSITQKKFLCDRTLWRIPFSSNSMSMGALTDLGQNLLYANSAHALDMT
FEVDPMDEPTLLYVLFEVSD
VVRVHRPSRGVIETVYLRTPFSAGNATT
**SEQ ID9**
   Mal d 1, malus domesticus, aminoacids 144-150
   FKLIESY
**SEQ ID10**
   Mal d 1, malus domesticus, F144S and Y150S mutations underlined (vegetal)
   SKLIESS
**SEQ ID11**
   Alpha-Gliadin (CD1d binding motif underlined) 
MVRVPVPQLQPQNPSQQQPQEQVPLVQQQFPGQQQPFPPQQPYPQPQPFPSQQPYL
QLQPFPQPQLPYPQPQLPY
PQPQLPYPQPQPFRPQQPYPQSQPQYSQPQQPISQQQQQQQQQQKQQQQQQQIL
QQILQQQLIPCRDVVLQQH
 
SIAYGSSQVLQQSTYQLVQQLCCQQLWQIPEQSRCQAIHNVVHAIILHQQQQQQQQ
QQQPLSQVSFQQPQQQYPS
GQGSFQPSQQNPQAQGSVQPQQLPQFEEIRNLALETLPAMCNVYIPPYCTIAPVGIFGT
NYR
**SEQ ID12**
   Alpha-Gliadin (mutation underlined) 
MVRVPVPQLQPQNPSQQQPQEQVPLVQQQFPGQQQPFPPQQPYPQPQPFPSQQPSL
QLQPFPQPQLPYPQPQLPY
PQPQLPYPQPQPFRPQQPYPQSQPQYSQPQQPISQQQQQQQQQQKQQQQQQQIL
QQILQQQLIPCRDVVLQQH
SIAYGSSQVLQQSTSQLVQQLCCQQLWQIPEQSRCQAISNVVHAIILHQQQQQQQ
QQQPLSQVSFQQPQQQYPS
GQGSFQPSQQNPQAQGSVQPQQLPQSEEIRNLALETLPAMCNVYIPPSCTIAPVGIFGT
NYR
**SEQ ID13**
   D. pteronyssinus Der p 1, aminoacids 38-44 (pyroglyphidae, Dermatophagoides pteronyssinus, European house dust mite)
   FSGVAAT
**SEQ ID14**
   D. pteronyssinus Der p 1, aminoacids 135-141 (pyroglyphidae, Dermatophagoides pteronyssinus, European house dust mite)
   HSAIAAVI
**SEQ ID15**
   D. pteronyssinus Der p 1, aminoacids 216-222 (pyroglyphidae, Dermatophagoides pteronyssinus, European house dust mite)
   YPYVVIL
**SEQ ID16**
   Mature Der p 1 (mutations of P1 anchoring residues F38S, H135S and Y216S underlined) (pyroglyphidae, Dermatophagoides pteronyssinus, European house dust mite) 
ETNACSINGNAPAEIDLRQMRTVTPIRMQGGCGSCWASSGVAATESAYLAYRNQSLD
LAEQELVDCASQHGCHGDTI
PRGIEYIQHNGVVQESYYRYVAREQSCRRPNAQRFGISNYCQIYPPNVNKIREALAQT
SSAIAVIIGIKDLDAFRHY
DGRTIIQRDNGYQPNYHAVNIVGYSNAQGVDYWIVRNSWDTNWGDNGYGYFAANI
DLMMIEESPYVVIL
**SEQ ID17**
   Mature Der p 1 (CD1d epitopes underlined): (pyroglyphidae, Dermatophagoides pteronyssinus, European house dust mite) 
ETNACSINGNAPAEIDLRQMRTVTPIRMQGGCGSCWAFSGVAATESAYLAYRNQSLD
LAEQELVDCASQHGCHGDTI
PRGIEYIQHNGVVQESYYRYVAREQSCRRPNAQRFGISNYCQIYPPNVNKIREALAQT
HSAIAVIIGIKDLDAFRHY
DGRTIIQRDNGYQPNYHAVNIVGYSNAQGVDYWIVRNSWDTNWGDNGYGYFAANI
DLMMIEEYPYVVIL
**SEQ ID18**
   IgG antibody, CH2 domain of IgG1 and IgG4 (human)
   YRVVSVL
**SEQ ID19**
   IgG antibody, CH2 domain of IgG2 and IgG3 (human)
   FRVVSVL
**SEQ ID20**
   IgG antibody, CH3 domain of IgG1, IgG2 and IgG4 (human)
   HEALHNH
**SEQ ID21**
   Human IgG4 FC fragment (CD1d epitopes underlined) (human) 
ASTKGPSVFPLAPCSRSTSESTAALGCLVKDYFPEPVTVSWNSGALTSGVHTFPAVLQ
SSGLYSLSSVVTVPSSSL
GTKTYTCNVDHKPSNTKVDKRVESKYGPPCPSCPAPEFLGGPSVFLFPPKPKDTLMIS
RTPEVTCVVVDVSQEDPE
VQFNWYVDGVEVHNAKTKPREEQFNSTYRVVSVLTVLHQDWLNGKEYKCKVSNK
GLPSSIEKTISKAKGQPREPQV
YTLPPSQEEMTKNQVSLTCLVKGFYPSDIAVEWESNGQPENNYKTTPPVLDSDGSFFL
YSRLTVDKSRWQEGNVFS
CSVMHEALHNHYTQKSLSLSLGK
**SEQ ID22**
   Human IgG4 FC fragment (mutated aminoacids underlined) (human)

ASTKGPSVFPLAPCSRSTSESTAALGCLVKDYFPEPVTVSWNSGALTSGVHTFPAVLQ
SSGLYSLSSVVTVPSSSL
GTKTYTCNVDHKPSNTKVDKRVESKYGPPCPSCPAPEFLGGPSVFLFPPKPKDTLMIS
RTPEVTCVVVDVSQEDPE
VQFNWYVDGVEVHNAKTKPREEQFNSTSRVVSVLTVLHQDWLNGKEYKCKVSNKG
LPSSIEKTISKAKGQPREPQV
YTLPPSQEEMTKNQVSLTCLVKGFYPSDIAVEWESNGQPENNYKTTPPVLDSDGSFFL
YSRLTVDKSRWQEGNVFS
CSVMSEALHNHYTQKSLSLSLGK

## Claims

1. A method to obtain a modified isolated peptide or polypeptide with reduced capacity to activate NKT cells comprising the steps of:
a. identification of at least one CD1d restricted NKT cell epitope within the natural amino acid sequence of said peptide wherein said epitope comprises the [FWTHY]-X₂X₃-[ILMV]-X₅X₆-[FWTHY] motif with hydrophobic amino acid residues in position P1 and/or P7
b. elimination of at least one CD1d-restricted NKT epitope by substituting or deletion of at least one hydrophobic aminoacid residue in position P1 and/or P7 with a non-hydrophobic residue or by addition of at least one amino acid in any location within said epitope.

2. The method according to claim 1 wherein elimination of said CD1d-restricted NKT cell epitope(s) occurs by substituting at least one hydrophobic aminoacid residue in position P1 and/or P7 with a non-hydrophobic residue.

3. The method according to claim 2, wherein at least one F, W, T, H, Y in position P1 and/or P7 is replaced by at least one any natural aminoacid, non-natural aminoacid or non-aromatic aminoacid compounds wherein said aminoacid is different from F, W, T, H, Y.

4. Method according to any of the preceding claims wherein elimination of said CD1d-restricted epitope(s) further includes substitution or deletion of an aliphatic residue in P4.

5. Method according to claim 4 **characterized in that** said aliphatic residue in P4 is I, L, or M.

6. An isolated polypeptide obtainable according to claims 1 to 5 and comprising SEQ.ID.NO:4.

## Patentansprüche

1. Verfahren zum Erhalten eines modifizierten isolierten Peptids oder Polypeptids mit verringerter Kapazität zum Aktivieren von NKT-Zellen, umfassend die Schritte:
a. Identifizieren von mindestens einem CD1d-eingeschränkten NKT-Zellepitop innerhalb der natürlichen Aminosäuresequenz des Peptids, wobei das Epitop das [FWTHY]-X₂X₃-[ILMV]-X₅X₆-[FWTHY]-Motiv mit hydrophoben Aminosäureresten in Position P1 und/oder P7 umfasst;
b. Eliminieren von mindestens einem CD1d-eingeschränkten NKT-Epitop durch Substitution oder Deletion von mindestens einem hydrophoben Aminosäurerest in Position P1 und/oder P7 durch einen nicht-hydrophoben Rest oder durch Addition von mindestens einer Aminosäure an einer beliebigen Stelle innerhalb des Epitops.

2. Verfahren nach Anspruch 1, wobei das Eliminieren von dem oder den CD1d-eingeschränkten NKT-Zellepitopen durch Substitution von mindestens einem hydrophoben Aminosäurerest in Position P1 und/oder P7 durch einen nicht-hydrophoben Rest erfolgt.

3. Verfahren nach Anspruch 2, wobei mindestens eine F, W, T, H, Y in Position P1 und/oder P7 durch mindestens eine beliebige natürliche Aminosäure, nicht-natürliche Aminosäure oder nicht-aromatische Aminosäureverbindung ersetzt wird, wobei die Aminosäure sich von F, W, T, H, Y unterscheidet.

4. Verfahren nach einem der vorhergehenden Ansprüche, wobei das Eliminieren des oder der CD1d-eingeschränkten Epitope weiterhin eine Substitution oder Deletion eines aliphatischen Rests in P4 umfasst.

5. Verfahren nach Anspruch 4, **dadurch gekennzeichnet, dass** der aliphatische Rest in P4 I, L oder M ist.

6. Isoliertes Polypeptid, das gemäß den Ansprüchen 1 bis 5 erhalten werden kann und SEQ ID Nr. 4 umfasst.

## Revendications

1. Une méthode pour l'obtention d'un peptide ou polypeptide isolé modifié avec une capacité réduite d'activation des cellules NKT comprenant les étapes de:
a. on identifie au moins un épitope de cellules NKT restreint par CD1d au sein de la séquence naturelle en acide aminés dudit peptide, dans laquelle ledit épitope comprend le motif [FWTHY]-X₂X₃-[ILMV]-X₅X₆-[FWTHY] avec des résidus hydrophobes à la position P1 et/ou P7
b. on élimine au moins un épitope de cellules NKT restreint par CD1d par substitution ou délétion d'au moins un acide aminé hydrophobe à la position P1 et/ou P7 par un résidu non hydrophobe ou par l'addition d'au moins un acide aminé à une quelconque localisation au sein dudit épitope.

2. La méthode selon la revendication 1 dans laquelle l'élimination dudit (desdits) épitope(s) de cellules NKT restreint(s) par CDld se fait par substitution d'au moins un acide aminé hydrophobe à la position P1 et/ou P7 par un résidu non hydrophobe.

3. La méthode selon la revendication 2 dans laquelle au moins un des F, W, T, H, Y à la position P1 et/ou P7 est remplacé par au moins un quelconque acide aminé naturel, acide aminé non naturel ou composés non aromatiques, dans laquelle ledit acide aminé est différent de F, W, T, H, Y.

4. La méthode selon une quelconque des revendications précédentes, dans laquelle l'élimination dudit (desdits) épitope(s) de cellules NKT restreint(s) par CDld inclut en outre la substitution ou délétion d'un résidu aliphatique en P4.

5. La méthode selon la revendication 4 **caractérisée en ce que** ledit résidu aliphatique en P4 est I, L, ou M.

6. Un polypeptide isolé susceptible d'être obtenu selon les revendications 1 à 5 et comprenant la SEQ.ID.NO:4.
